# EUROPEAN PATENT SPECIFICATION

(11) **EP 3 801 452 B1**
(45) Date of publication and mention of the grant of the patent: **13.04.2022**
(21) Application number: 19728072.0
(22) Date of filing: 05.06.2019
(51) Int. Cl.: A61K 8/26, A61K 8/73, A61K 8/04, A61K 9/00, A61K 9/70, A61Q 17/00, A61Q 17/04, A61K 8/02

(54) **THIXOTROPIC COMPOSITION**
THIXOTROPE ZUSAMMENSETZUNG
COMPOSITION THIXOTROPE

(30) Priority: 05.06.2018 EP 18175964
(43) Date of publication of application: 14.04.2021
(73) Proprietor: Bio-Gate AG, 90411 Nürnberg (DE)
(72) Inventor: STEINRÜCKE, Peter, 90461 Nürnberg (DE); HEINAR, Thomas, Toronto, Ontario M1M 1S6 (CA)
(74) Representative: Dr. Gassner & Partner mbB
(86) International application number: PCT/EP2019/064605
(87) International publication number: WO 2019/234079

(56) References cited:
- WO-A1-2013/052114
- WO-A2-2004/026263
- KR-B1- 100 786 492
- US-A1- 2008 226 717
- US-A1- 2014 302 125

## Description

The invention concerns a thixotropic composition comprising a microcrystalline cellulose, a solvent and carboxymethyl cellulose. Microcrystalline cellulose is partly depolymerized cellulose usually derived from wood pulp. Carboxymethyl cellulose is also designated as cellulose gum.

From WO 2013/052114 A1 a stabilizer composition of microcrystalline cellulose and carboxymethyl cellulose is known. This composition is useful as a stabilizer in edible food products. The composition may comprise water and modified starch. It is disclosed that colloidal microcrystalline cellulose being dispersed in water forms thixotropic gels.

US 2011/0143009 A1 discloses compositions comprising microcrystalline cellulose, cellulose ether, and salt. The compositions can act as stabilizers that have a multitude of industrial and consumer uses, for example, in the food and beverage industry, or in suspensions for industrial application.

US 2014/0302125 A1 relates to a once-a-day therapeutically synergistic pharmaceutical dosage form for treatment of cardiovascular disorders. According to example 1 microcrystalline cellulose spheres were given seal coat I of ethyl cellulose. These seal coated pellets were subjected to metoprolol succinate layering with a binder in aqueous solvent system. Drug layered pellets were provided with an extended release coating-I using ethyl cellulose and opadry^{®}. An extended release coating-II of eudragit^{®} was given using plasticizer, triethyl citrate and talc. Seal coat-II was given to extended release coated pellets followed by PEG coating in suitable solvent system. These PEG coated pellets was blended with the Prosolv^{®}, amlodipine besylate, croscarmellose sodium, PEG and Sodium Stearyl Fumarate and compressed into a tablet. An opadry^{®} coat was given to core tablets.

KR 100 786 492 B1 is directed to a thixotropic gel type cosmetic composition comprising a cellulose mixture consisting of microcrystalline cellulose and cellulose gum, wherein the content of the cellulose mixture regarding the total weight of the cosmetic composition is 0.05-10 wt.% and the remaining consists of purified water.

WO 2004/026263 A2 discloses a sprayable cosmetic composition. In one aspect, the composition comprises a sunscreen agent or mixture of sunscreen agents, an emulsifier or mixture of emulsifiers, optionally an emollient or mixture of emollients, a rheology control agent and water. The rheology control agent is microcrystalline cellulose having an average particle size of 50 microns or less. The microcrystalline cellulose may be a coprocessed microcrystalline cellulose that comprises microcrystalline cellulose and sodium carboxymethylcellulose. The coprocessed microcrystalline cellulose may comprise the microcrystalline cellulose and the sodium carboxymethylcellulose in a ratio of about 85/15, by weight.

US 2008/226717 A1 relates to a sublingual coated tablet consisting of a compressed core devoid of pharmaceutically active substance and comprising one or more diluting agents, and a coating comprising an active substance. The diluting agent is chosen from a group comprising in particular cellulose derivatives, and preferably microcrystalline cellulose, polyols, starches alone, and sugar derivatives. The diluting agent can be chosen from sucrose, lactose, fructose, dextrose, mannitol, sorbitol, lactitol, erythritol, xylitol, dicalcium phosphate, tricalcium phosphate or a microcrystalline cellulose, alone or as a mixture. In an embodiment, the compressed core comprises a mixture of diluting agent formed from mannitol and microcrystalline cellulose. The swelling agent may be chosen from the group comprising microcrystalline cellulose, starches, modified starches, such as carboxymethyl starch or sodium starch glycolate, alginic acid or sodium alginate, and mixtures thereof.

US 7,232,481 B1 concerns a method for producing an anti-swelling mica. According to US 7,232,481 B1 a nanocomposite produced from an aluminosillicate (3Al₂O₃.3SiO₂) such as clay minerals (montmorillonite) and a mica is a common technique. Generally, the mica is swelling, but a swelling mica is not practical. The swelling mica is only used as a packing material in semiconductor industry. WO 2004/048267 A1 discloses a synthesis method for swelling Na-4-mica. In particular WO 2004/048267 A1 concerns a synthesis method for highly charged sodium fluorophlogopite (Na-4-mica) pure product and more particularly for phasepure Na-4-mica, which exhibits exceptionally high uptake ability and selectivity for harmful radioactive and heavy metal cations as well as high stability.

The problem to be solved by the present invention is to provide a thixotropic composition that can be diluted over the wide range without loss of stability, that is relative stable over a relative long period of time and that is sprayable at least if it is diluted. Furthermore, the thixotropic composition shall be able to suspend particles of high density, keep these particles suspended and keep this suspension stable over a relative long period of time. "Stable" means that no or only little separation of water and no or only little precipitation of the particles of high density occurs over a relative long period of time, such as two days, two months, six months, one year, two years, three years or even five years. Furthermore, a device comprising the thixotropic composition and a use of the thixotropic composition shall be provided.

The invention is defined as in claims.

The problems are solved by the features of claims 1, 17 and 18. Embodiments are subject matter of claims 2 to 16.

According to the invention a thixotropic composition is provided, which thixotropic composition comprises a microcrystalline cellulose, a swelling or water binding agent, a starch glycolate, a solvent and carboxymethyl cellulose or its alkaline salt or its alkaline earth metal salt, wherein the swelling or water binding agent is a mica, wherein the solvent comprises or consists of water. The water content in the composition should maybe at least 70% w/w, wherein the weight ratio of microcrystalline cellulose to carboxymethyl cellulose or its alkaline salt or its alkaline earth metal salt is in the range of 3 : 1 to 11 : 1 and/or the weight ratio of microcrystalline cellulose and carboxymethyl cellulose or its alkaline salt or its alkaline earth metal salt together to the swelling or water binding agent is in the range of 3 : 1 to 4 : 1. The water content in the composition may be in particular at least 75% w/w, in particular at least 80% w/w. In a further embodiment the water content in the composition is at least 85% w/w. The swelling or water binding agent may be present in a concentration of 0.1% w/w to 5% w/w of the total thixotropic composition. The starch glycolate may be present in a concentration of 0.01% w/w to 4% w/w of the total thixotropic composition. The remaining percentage may be provided by microcrystalline cellulose together with the carboxymethyl cellulose or its alkaline salt or its alkaline earth metal salt, optionally together with further ingredients, in particular further ingredients mentioned below, in particular further ingredients mentioned below in the concentrations mentioned below. In particular, the microcrystalline cellulose together with the carboxymethyl cellulose or its alkaline salt or its alkaline earth metal salt may be present in a total concentration of at least 0.2% w/w, in particular 0.3% w/w to 31% w/w, in particular 0.4% w/w to 20% w/w, in particular 0.5% w/w to 15% w/w, of the total thixotropic composition. Phyllosilicates are silicates formed of parallel sheets of silicate tetrahedra which sheets are not linked via Si-O bonds. H₂O molecules and big cations can be incorporated between the sheets. The phyllosilicate in the thixotropic composition according to the invention may be a gel forming substance. The phyllosilicate is mica, in particular a dioctahedral mica such as muscovite, or a trioctahedral mica such as biotite, lepidolite or phlogopite. The mica may be a natural or a synthetic mica, in particular a nature identical synthetic mica. The mica may be in the form of a powder, e. g. a powder obtained by grinding of natural or synthetic mica. The particles in the powder may be in the form of short fibers. The mica in the form of a powder advantageously influences rheological behavior of the thixotropic composition according to the invention. The mica may be mica having CAS Registry Number 12001-26-2. In an embodiment the mica is an aluminum fluoro magnesium sodium silicate, in particular aluminum fluoro magnesium sodium silicate having CAS Registry Number 12001-26-2. In an embodiment the mica is Submica^{®} E from Sensient Technologies Corporation, 777 East Wisconsin Avenue, Milwaukee, WI 53202-5304 USA.

The thixotropic composition according to the invention has very advantageous features that are owing to the combination of the specific swelling or water binding agent, the starch glycolate and the microcrystalline cellulose. When a known gel formed of microcrystalline cellulose and a solvent is moved, e. g. by stirring, the solvent is squeezed out of the gel. After stopping of movement the solvent is taken up by the gel only after a period of time, if at all. This results in a low stability of such a gel and in the precipitation of particles of high density suspended in such a gel during the squeeze out of the solvent. In contrast to this no free solvent becomes present when the thixotropic composition according to the invention is moved. It is assumed that solvent squeezed out from the gel formed by the microcrystalline cellulose is taken up by the swelling or water binding agent and the starch glycolate rapidly and over time moves back from the swelling or water binding agent and the starch glycolate to the gel. The phyllosilicate can bind and release water very quickly. Furthermore, there is no or nearly no separation of the solvent even if the composition according to the invention is stored for a relative long period of time. This makes it an ideal base for a preparation of cosmetics or medical creams which can be stored for a relative long time without or nearly without separation of its components. The starch glycolate may be an alkaline starch glycolate, in particular sodium starch glycolate.

A further feature of the composition according to the invention is that it can be prepared in a highly concentrated form, i. e., in a form having a low content of solvent, which highly concentrated form can be highly diluted without any phase separation of the composition or precipitation of particles suspended therein. Even if the particles are particles having a high density such as metal particles, in particular particles of heavy metal, a highly concentrated composition according to the invention can be highly diluted without precipitation of these particles. This is also true when the particles have a rough surface such as porous metal particles which highly tend to aggregate and to precipitate. This enables sales and distribution of a highly concentrated composition according to the invention as basis for the preparation of cosmetics or medical creams. Such a concentrated composition makes it much easier for manufacturers to produce the cosmetics or medical creams since the problem of preparing a stable suspension of the particles having a high density is omitted.

The composition according to the invention is stable in a pH range of at least 4.5 to 9.5. This is also advantageous for the preparation of cosmetics and medical creams because the usual pH range of cosmetics and medical creams is within this range.

Furthermore, the composition according to the invention can be sprayed or nebulized very well if it contains at least 85% w/w of water, in particular at least 90% w/w of water. The reason for that is that relative low input of energy results in a relative high liquidation of the composition which high liquidation results in the generation of relative small droplets during spraying or nebulizing and an even spray pattern resulting in a uniform covering of a relative large area of a surface on which the composition is sprayed. Immediately after being sprayed on the surface the composition becomes sticky. As a result, the composition sprayed on a surface does not tend to flow away from this surface even if the surface is vertically orientated and even if a bigger amount of this composition is sprayed on the surface. Excellent spraying results can be achieved with the composition according to the invention. This makes the composition according to the invention an ideal base for the preparation of a paint or a lacquer or an impregnant for impregnating textiles by spraying. The textiles may be woven fabrics or non-woven fabrics. If the composition comprises particles suspended therein, spraying of the composition on a surface results in a uniform distribution of the particles on the surface.

Owing to the low input of energy required for liquidation of the composition the device for spraying the composition may be designed as a pump spray. The energy can be provided, e. g., by shaking or by suction into an actuator of the pump spray. Some of the advantages of pump spray devices are that they are cheap in production, require no specific safety precautions for shipping and are allowed in airplane cabins. If the composition is of low viscosity a liquefied gas, such as butane, can be used as a propellant. In this case the gas can be in direct contact with the composition or the composition can be sprayed by use of a bag on valve system. A typical droplet size that can be achieved by use of a pump spray or by a pressure gas driven spraying process or by nebulizing in a medical nebulizing device is in the range of 1 µm to 140 µm, in particular 2 µm to 130 µm. The droplet size achievable by spraying or nebulizing allows a medical application of the composition by inhalation. For this purpose the composition can be formulated as a medicament, e. g., for use in the treatment of a pulmonary disorder or pulmonary disease.

The sprayable composition according to the invention may also be, e. g., a body care product or a sunscreen to be sprayed on skin, a medicament to be sprayed on skin or to be nebulized and inhaled, a wound treatment product to be sprayed on a wound, or a paint, lacquer, impregnant or any other coating to be sprayed on a surface for coating that surface.

Furthermore, the stability of the composition according to the invention makes it well suited for the application on textiles by pulling them through the composition, such as in a foulard process. The application on textiles may be an application for providing the textile with specific properties, such as being antimicrobial by application of a composition according to the invention comprising particles of metallic silver. The textile may be a woven fabric or a non-woven fabric.

In the thixotropic composition according to the invention the weight ratio of microcrystalline cellulose to carboxymethyl cellulose or its alkaline salt or its alkaline earth metal salt is in the range of 3 : 1 to 11 : 1, in particular 4 : 1 to 9 : 1. The weight ratio of microcrystalline cellulose and carboxymethyl cellulose or its alkaline salt or its alkaline earth metal salt together to the swelling or water binding agent is in the range of 3 : 1 to 4 : 1, in particular 3.3 : 1 to 3.5 : 1. These ratios have been found to result in particular stable compositions according to the invention. The thixotropic composition according to the invention may further comprise a cellulose or an alkylcellulose, in particular methylcellulose, ethylcellulose, or ethyl methyl cellulose. These compounds can further bind water and thereby further stabilize the thixotropic composition.

In an embodiment of the invention a dye and/or chemical substance is dissolved in the thixotropic composition. The chemical substance may be a pesticide, a drug or an insect repellent. In addition or in another embodiment pigments, any particles having a diameter in the range of 1 to 100 µm, metal particles, nanocapsules and/or microcapsules may be suspended in the thixotropic composition. Nanocapsules are capsules having a diameter of at least 1 nm and below 1 µm. Microcapsules are capsules having a diameter of at least 1 µm and below 1 mm. For the dye, the chemical substance, the pigments, the particles having a diameter in the range of 1 to 100 µm, the metal particles, the nanocapsules and the microcapsules the advantage achieved in combination with the thixotropic composition according to the invention is that the thixotropic composition enables a very even distribution of these substances on a surface by spraying. The combination of a dye or pigments with the chemical substance may be useful because the dye or pigments can work as indicator allowing to recognize a surface area to which the thixotropic composition has been applied. The metal particles may be particles that have an optical effect in a thixotropic composition according to the invention formulated as a paint or a lacquer. Also the thixotropic composition comprising the dye and/or the pigments may be formulated as a paint or a lacquer.

The material encapsulated by the nanocapsules and the microcapsules may be any organic or inorganic material having an antimicrobial effect, i. e. an effect which reduces the number of microbes, such as bacteria or fungi, at the site of application. The encapsulated material may also comprise or consist of metal particles, in particular particles of metallic silver, an oxygen sensitive compound, a photosensing compound, a pH value indicating compound, a vitamin, a drug, a cosmetic compound, a thermochromic indicator, a compound for the treatment of a textile, e. g. for impregnating the textile, or a volatile compound, such as a fragrance. The material forming the capsule around the encapsulated material may be shellac, a polymer or a blend of polymers, a wax or a blend of waxes, a fat or a blend of fats, a fatty acid or a blend of fatty acids or a surfactant or a blend of surfactants. The polymer may be a polymer from a biological source, such as vegetable, animal or bacterial source or a synthetic polymer. Examples of a polymer from a biological source are alginates, pectins, chitosans, carrageenans, arabic gums, cellulose derivatives, starches, gelatins, milk proteins and gellan gum. Examples of a synthetic polymer are poly(vinyl acetate), polyvinyl alcohol, polyethylene glycol, polycaprolactone, poly(lactic-co-glycolic acid), isocyanates, polyamide, polyurea, polyurethane and melamine formaldehyde. The wax, the fatty acid and the fat may be of vegetable or animal source. Examples of a wax are candelilla wax and carnauba wax. Examples of a fatty acid are stearic and palmitic acid. The blend of fatty acids may be stearin. Examples of a surfactant are a lecithin, a sorbitan and a polysorbate.

In an embodiment the metal particles are particles of a heavy metal, in particular copper, silver or zinc. According to the invention a heavy metal is a metal having a density above 5 g/cm³, in particular above 7 g/cm³. Such particles may exert an antimicrobial effect by releasing antimicrobial metal ions. With respect to a biological, in particular antimicrobial effect, of metal ions, in particular of silver ions, released from the metal particles, the inventors found that the phyllosilicate, in particular the mica, has not only an advantageous effect with respect to thixotropy but also a buffering effect on these ions. The ions can be bound and released by the phyllosilicate, in particular by the mica.

The metal particles may be particles of silver having a diameter between 1 to 100 µm and an arithmetic mean inner porosity of at least 65%, in particular between 65% and 90% or 95% such as those described in WO 2005/023213. Since these particles have at high tendency to aggregate when being in known suspensions, the possibility to prevent aggregation for a relative long period of time by suspending these particles in the thixotropic composition according to the invention enables new applications such as providing long term stable suspensions of such metal particles capable of being sprayed. The arithmetic average diameter of the particles may be in the range of 10 µm to 90 µm.

Inner porosity is understood as meaning the percentage of the volume of the particle which is not filled with metal. The mean inner porosity of the particles can be determined using the following method:
1. Embedding the particles in a plastic,
2. preparing ultrathin sections of the embedded particles,
3. taking transmission electron microscopic (TEM) photographs of the sections of the particles,
4. determining the percentage of the area within each particle which is not filled with metal, in relation to the total area of this particle, in a plurality of TEM photographs, and
5. calculating the mean of a plurality of percentages which have been determined in this way.

In this connection, step 4 can be effected by means of a computer-assisted image analysis of the TEM photographs.

The metal particles may be present in the thixotropic composition according to the invention in a concentration range of 0.005% w/w to 0.5% w/w, in particular in a concentration range of 0.05% w/w to 0.2% w/w, in particular in a concentration range of 0.1% w/w to 0.2% w/w.

The metal particles may be encapsulated metal particles or metal particles having an optical effect by having a ratio of surface area to volume of at least 5, in particular at least 10, in particular at least 15, in particular at least 20.

In an embodiment the solvent comprises or consists of water and a diol, in particular a butanediol or a propanediol, in particular propane-1,2-diol, a triol, in particular glycerol, or a polyol. If heavy metal particles and in particular porous heavy metal particles shall be suspended in the thixotropic composition according to the invention, an easy way to suspend the particles in the thixotropic composition is suspending them in the diol, triol or polyol and then mixing the resulting suspension with the other components of the thixotropic composition. The diol may be present in a concentration of 0.1% w/w to 5% w/w of the total thixotropic composition.

That thixotropic composition may have a water content of at least 85% w/w. If that thixotropic composition shall be prepared as a concentrate a content of water in the composition in a range of 80% w/w to 90% w/w is appropriate. The diluted concentrate or the thixotropic composition prepared with its final concentrations of its constituents may have a water content of at least 95% w/w, in particular 95% w/w to 98% w/w. By choosing an appropriate water content viscosity of the thixotropic composition can be adapted to a desired use such as in a desired spraying device. In this case the water content is chosen such that the thixotropic composition is well sprayable with this device.

The crystalline cellulose may be partly depolymerized microcrystalline cellulose having CAS Registry Number 9004-34-6.

In an embodiment of the thixotropic composition according to the invention the alkali salt of the carboxymethyl cellulose is sodium carboxymethyl cellulose, in particular sodium carboxymethyl cellulose having CAS Registry Number 9004-32-4. The alkaline earth metal salt of the carboxymethyl cellulose may be calcium carboxymethyl cellulose or magnesium carboxymethyl cellulose.

The thixotropic composition according to the invention can be formulated as a a body care product, a sunscreen, a medicament, a spray for plants, a paint, a lacquer, in particular a colorless lacquer, an impregnant or another coating to be applied on a surface for coating that surface. The body care product can be a skin care product. The medicament can be a medicament for use in the treatment of wounds to achieve or to improve healing of the wounds or a medicament for use in the treatment of a dermatological disorder or disease. Any body care product and any medicament mentioned herein can be for human or for animal use.

When coating surfaces with a paint, a lacquer or an impregnant and the surfaces are not horizontally orientated, a common problem is the formation of tears. The tendency of the thixotropic composition according to the invention to form tears when applied on a non-horizontally orientated surface is very low. Therefore, the thixotropic composition according to the invention is very suitable to be formulated and used as a paint, a lacquer, an impregnant or another coating to be applied on a surface for coating that surface, in particular a paint, a lacquer, an impregnant or another coating to be applied by spraying.

The invention further concerns a spraying device containing the thixotropic composition according to the invention. The content of water of the thixotropic composition in the spraying device is at least 85%. In particular it may be at least 90%, in particular at least 95%.

The invention further concerns a use of the thixotropic composition according to the invention for the preparation of a medical cream, a wound treatment product, a body care product, a sunscreen or a cosmetic, in particular a use of the thixotropic composition according to the invention as a basis for the preparation of a medical cream, a wound treatment product, a body care product, a sunscreen or a cosmetic. During this use further ingredients for providing the medical, wound treating, body caring, sun protective or cosmetic effect are usually mixed with the thixotropic composition according to the invention.

In the following the invention is illustrated by means of examples for demonstrating which thixotropic compositions are able to stably suspend porous particles of silver, to be diluted with water to an appropriate silver concentration, and to produce an acceptable spray pattern under controlled conditions.
Figure 1 shows a spray pattern of thixotropic composition 216-1D according to the invention.
Figure 2 shows a spray pattern of thixotropic composition 216-2D not covered by the invention.
Figure 3 shows a spray pattern of thixotropic composition 216-3D not covered by the invention.
Figure 4 shows a spray pattern of thixotropic composition 216-4D not covered by the invention.
Figure 5 shows a spray pattern of thixotropic composition 248-2D not covered by the invention.
Figure 6 shows a spray pattern of thixotropic composition 248-3D not covered by the invention.
Figure 7 shows a spray pattern of thixotropic composition 248-4D not covered by the invention.
Figure 8 shows a spray pattern of thixotropic composition 248-5D according to the invention.
Figure 9 shows a spray pattern of water as a control.

Concentrated thixotropic compositions comprising porous silver particles having a diameter in the range of 1 to 100 µm and an arithmetic mean inner porosity of at least 65% (MicroSilver BG, Bio-Gate AG, Neumeyerstraße 28-34, 90411 Nuremberg, Germany) were prepared as base formulations and then diluted with water to achieve a concentration of silver of 0.1% w/w in each case.

For determining stability the resulting suspensions were left to stand for 48 hours and the degree of visible settling was determined and measured.

For determining spray patterns a bottle with dip tube and spray actuator was filled with one of the diluted suspensions in each case or with water as control.

In each case this bottle was placed before a vertical panel which was prepared to reveal the spray pattern of one pump of the actuator only from a distance of 160 mm. The non-reflective black surface of the panel was coated with a hygroscopic white powder evenly with a metal straight edge. The resultant spray droplets were allowed to stand for 2 minutes and then the loose hygroscopic powder was carefully brushed away revealing the coagulated droplet pattern which was then measured and photographed form a distance of 290 mm.

The following thixotropic compositions were prepared:
**Concentrated composition:**
**216-1C**

**Ingredients:**

| | |
|---|---|
| □ Water | 85.85% |
| □ Natpure Cellgum Plus^{®} (Microcrystalline Cellulose, Cellulose Gum) | 8.50% |
| □ Submica^{®} E (Mica) | 2.50% |
| □ Zemea^{®} (Propanediol) | 2.50% |
| □ Silver (MicroSilver BG^{™}) | 0.50% |
| □ Sodium Starch Glycolate | 0.15% |
| **Total:** | 100.00% |

**Diluted composition:**
**216-1D**

**Ingredients:**

| | |
|---|---|
| □ Water | 97.17% |
| □ Natpure Cellgum Plus^{®} | 1.70% |
| □ Submica^{®} E | 0.50% |
| □ Zemea^{®} | 0.50% |
| □ MicroSilver BG^{™} | 0.10% |
| □ Sodium Starch Glycolate | 0.03% |
| **Total:** | 100.00% |

| | |
|---|---|
| □ Level (mm) of water separation: from top / total height: **none** □ Product consistency: **uniform** □ Spray pattern (mm): - Width: **150 mm** - Height: **140 mm** | |

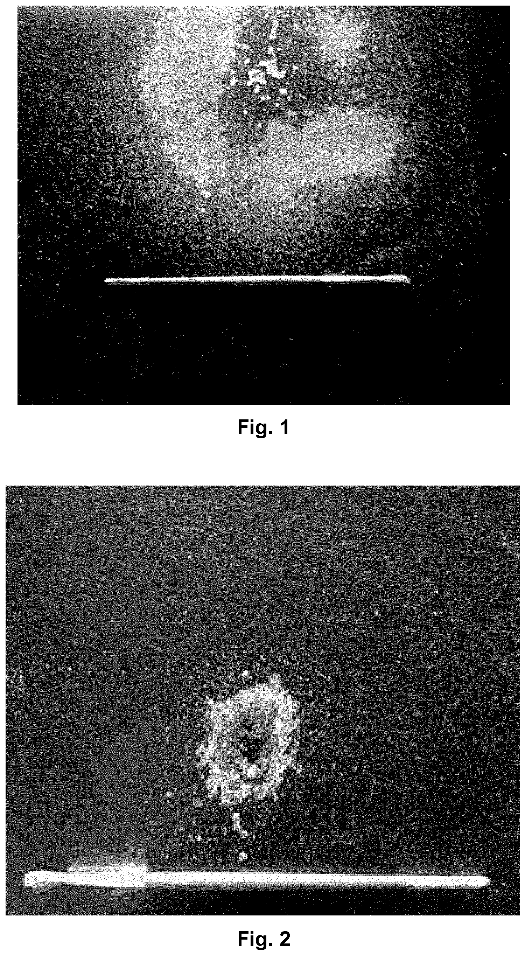

The spray pattern obtained with composition 216-1D is shown in Fig. 1. In this and all other figures the brush that can be seen below the spray pattern is used as reference having a length of 170 mm.
**Concentrated composition:**
**216-2C** (not in accordance with the invention) ***Ingredients:***

| | |
|---|---|
| □ Water | 86.00% |
| □ Natpure Cellgum Plus^{®} (Microcrystalline Cellulose, Cellulose Gum) | 8.50% |
| □ Submica^{®} E (Mica) | 2.50% |
| □ Zemea^{®} (Propanediol) | 2.50% |
| □ Silver (MicroSilver BG^{™}) | 0.50% |
| **Total:** | 100.00% |

**Diluted composition:**
**216-2D** (not in accordance with the invention) ***Ingredients:***

| | |
|---|---|
| □ Water | 97.20% |
| □ Natpure Cellgum Plus^{®} | 1.70% |
| □ Submica^{®} E | 0.50% |
| □ Zemea^{®} | 0.50% |
| □ MicroSilver BG^{™} | 0.10% |
| **Total:** | 100.00% |

| | |
|---|---|
| □ Level (mm) of water separation: from top / total height: **8 mm / 55 mm** □ Product consistency: **thin** □ Spray pattern (mm): - Width: **60 mm** - Height: **50 mm** | |

The spray pattern obtained with composition 216-2D is shown in Fig. 2.
**Concentrated composition:**
**216-3C**

**Ingredients:**

| | |
|---|---|
| □ Water | 88.35% |
| □ Natpure Cellgum Plus^{®} (Microcrystalline Cellulose, Cellulose Gum) | 8.50% |
| □ Zemea^{®} (Propanediol) | 2.50% |
| □ Silver (MicroSilver BG^{™}) | 0.50% |
| □ Sodium Starch Glycolate | 0.15% |
| **Total:** | 100.00% |

**Diluted composition:**
**216-3D**

**Ingredients:**

| | |
|---|---|
| □ Water | 97.67% |
| □ Natpure Cellgum Plus^{®} | 1.70% |
| □ Zemea^{®} | 0.50% |
| □ MicroSilver BG^{™} | 0.10% |
| □ Sodium Starch Glycolate | 0.03% |
| **Total:** | 100.00% |

| | |
|---|---|
| □ Level (mm) of water separation: from top / total height: **10 mm / 55 mm** □ Product consistency: **thin** □ Spray pattern (mm): - Width: **140 mm** - Height: **190 mm** | |

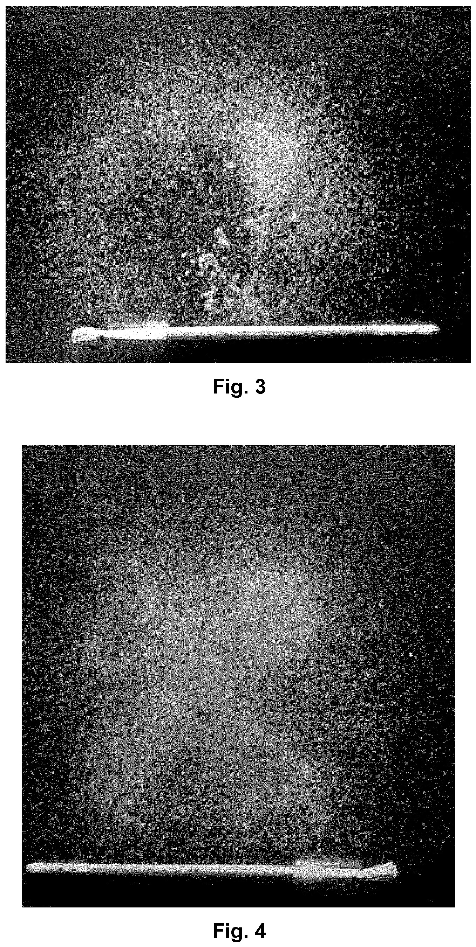

The spray pattern obtained with composition 216-3D is shown in Fig. 3.
**Concentrated composition:**
**216-4C**
(not in accordance with the invention) ***Ingredients:***

| | |
|---|---|
| □ Water | 88.50% |
| □ Natpure Cellgum Plus^{®} (Microcrystalline Cellulose, Cellulose Gum) | 8.50% |
| □ Zemea^{®} (Propanediol) | 2.50% |
| □ Silver (MicroSilver BG^{™}) | 0.50% |
| **Total:** | 100.00% |

**Diluted composition:**
**216-4D**
(not in accordance with the invention) ***Ingredients:***

| | |
|---|---|
| □ Water | 97.70% |
| □ Natpure Cellgum Plus^{®} | 1.70% |
| □ Zemea^{®} | 0.50% |
| □ MicroSilver BG^{™} | 0.10% |
| **Total:** | 100.00% |

| | |
|---|---|
| □ Level (mm) of water separation: from top / total height: **10 mm / 55 mm** □ Product consistency: **thin** □ Spray pattern (mm): - Width: **130 mm** - Height: **150 mm** | |

The spray pattern obtained with composition 216-4D is shown in Fig. 4.
**Further diluted compositions:**
(not in accordance with the invention) **248-2D**

**Ingredients:**

| | |
|---|---|
| □ Water | 97.17% |
| □ JRS Vivapur^{®} CS 032 XV (Microcrystalline Cellulose, Xanthan Gum) | 1.70% |
| □ Submica^{®} E | 0.50% |
| □ Zemea^{®} | 0.50% |
| □ MicroSilver BG^{™} | 0.10% |
| □ Sodium Starch Glycolate | 0.03% |
| **Total:** | 100.00% |

| | |
|---|---|
| □ Level (mm) of water separation: from top / total height: **none** □ Product consistency: **viscous** □ Spray pattern (mm): - Width: **85 mm** - Height: **80 mm** | |

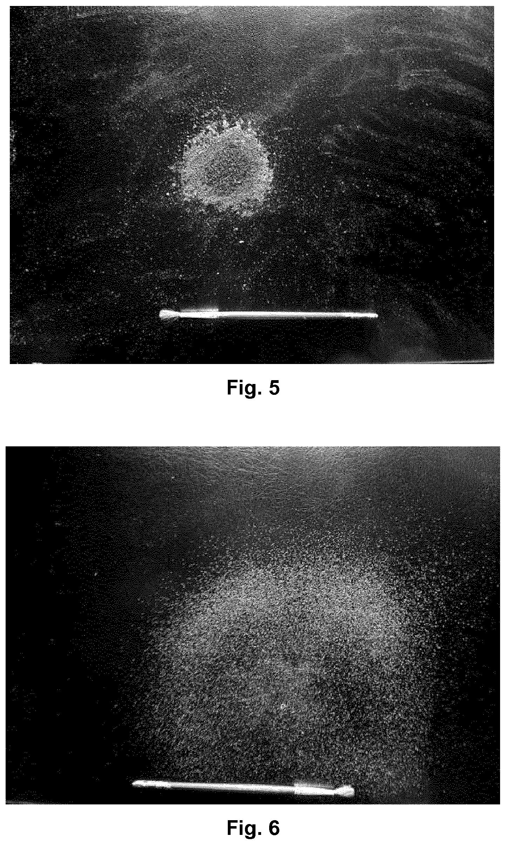

The spray pattern obtained with composition 248-2D is shown in Fig. 5. (not in accordance with the invention) **248-3D**

**Ingredients:**

| | |
|---|---|
| □ Water | 97.17% |
| □ JRS Vitacel^{®} CS 200 FC (Cellulose) | 1.70% |
| □ Submica^{®} E | 0.50% |
| □ Zemea^{®} | 0.50% |
| □ MicroSilver BG^{™} | 0.10% |
| □ Sodium Starch Glycolate | 0.03% |
| **Total:** | 100.00% |

| | |
|---|---|
| □ Level (mm) of water separation: from top / total height: **60 mm / 70 mm** □ Product consistency: **thin** □ Spray pattern (mm): - Width: **235 mm** - Height: **190 mm** | |

The spray pattern obtained with composition 248-3D is shown in Fig. 6. (not in accordance with the invention) **248-4D**

**Ingredients:**

| | |
|---|---|
| □ Water ................................................. | 97.17% |
| □ Seppic^{™} Solugum^{™} TARA (Caesalpinia Spinosa Gum) ........................ | 1.70% |
| □ Submica^{®} E.............................................................................................. | 0.50% |
| □ Zemea^{®}.................................................................................................... | 0.50% |
| □ MicroSilver BG^{™} ..................................................................................... | 0.10% |
| □ Sodium Starch Glycolate ......................................................................... | 0.03% |
| **Total:** | 100.00% |

| | |
|---|---|
| □ Level (mm) of water separation: from top / total height: **none** □ Product consistency: **viscous gel** □ Spray pattern (mm): - Width: **12 mm** - Height: **30 mm** | |

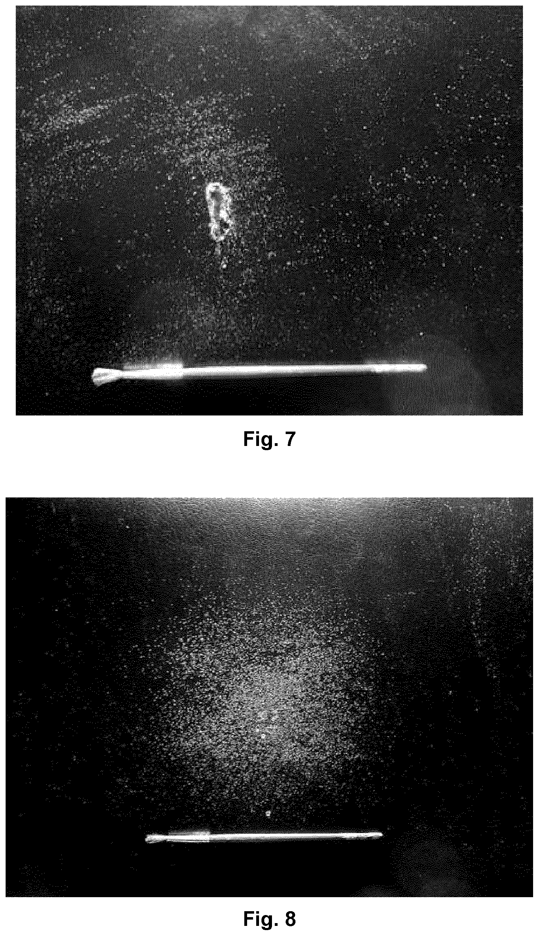

The spray pattern obtained with composition 248-4D is shown in Fig. 7.
**248-5D**

**Ingredients:**

| | |
|---|---|
| □ Water ..................................................................................................... | 97.17% |
| □ JRS Vivapur^{®} COS 8 (Microcrystalline Cellulose, Cellulose gum)........... | 1.70% |
| □ Submica^{®} E.............................................................................................. | 0.50% |
| □ Zemea^{®}.................................................................................................... | 0.50% |
| □ MicroSilver BG^{™} ..................................................................................... | 0.10% |
| □ Sodium Starch Glycolate ......................................................................... | 0.03% |
| **Total:** | 100.00% |

| | |
|---|---|
| □ Level (mm) of water separation: from top / total height: **none** □ Product consistency: **viscous gel** □ Spray pattern (mm): - Width: **180 mm** - Height: **160 mm** | |

The spray pattern obtained with composition 248-5D is shown in Fig. 8.

### Water Control

| □ Spray pattern (mm): | |
|---|---|
| - Width: | **200 mm** |
| - Height: | **145 mm** |

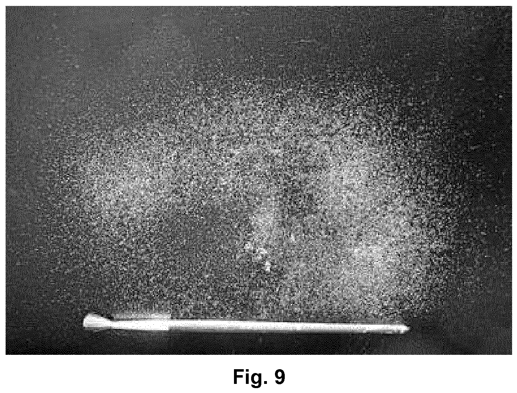

The spray pattern obtained with the water control is shown in Fig. 9.

The spray pattern of the water control and samples 216-1D and 248-5D produced a desirable distribution of droplets from one actuation of the spray pump. It can be concluded that the stability indicated by no visible separation of water goes along with a desired relatively wide and relatively uniform distribution of the spray pattern droplets such as in samples 216-1D and 248-5D. Sample 216-2D only differs from sample 216-1D by the absence of sodium starch glycolate which absence is compensated by a slightly higher amount of water. It shows water separation and a narrow spray pattern and therewith the importance of the starch glycolate. The results also show that concentrated thixotropic compositions according to the invention comprising heavy metal particles can be diluted such that the silver particles are suspended uniformly and present in low concentrations in compositions of low viscosity that can be effectively sprayed with a wide and uniform pattern resulting in a consistent distribution of the silver particles to a surface. The results further show that omitting one of the ingredients starch glycolate, carboxymethyl cellulose and phyllosilicate results in an undesired spray pattern and/or in water separation.

## Claims

1. Thixotropic composition comprising a microcrystalline cellulose, a swelling or water binding agent, a starch glycolate, a solvent, and carboxymethyl cellulose or its alkaline salt or its alkaline earth metal salt, wherein the swelling or water binding agent is mica, wherein the solvent comprises or consists of water, wherein a water content of the composition is at least 70% w/w, wherein the weight ratio of microcrystalline cellulose to carboxymethyl cellulose or its alkaline salt or its alkaline earth metal salt is in the range of 3 : 1 to 11 : 1 and/or the weight ratio of microcrystalline cellulose and carboxymethyl cellulose or its alkaline salt or its alkaline earth metal salt together to the swelling or water binding agent is in the range of 3 : 1 to 4 : 1.

2. Thixotropic composition according to claim 1, wherein water content of the composition is at least 80% w/w.

3. Thixotropic composition according to claim 1 or 2, wherein the mica is mica having CAS Registry Number 12001-26-2.

4. Thixotropic composition according to claim 1 or 2, wherein the mica is an aluminum fluoro magnesium sodium silicate.

5. Thixotropic composition according to any of the preceding claims, wherein the weight ratio of microcrystalline cellulose to carboxymethyl cellulose or its alkaline salt or its alkaline earth metal salt is in the range of 4 : 1 to 9 : 1 and/or the weight ratio of microcrystalline cellulose and carboxymethyl cellulose or its alkaline salt or its alkaline earth metal salt together to the swelling or water binding agent is in the range of 3.3 : 1 to 3.5 : 1.

6. Thixotropic composition according to any of the preceding claims, wherein the thixotropic composition further comprises a cellulose or an alkylcellulose, in particular methylcellulose, ethylcellulose, or ethyl methyl cellulose.

7. Thixotropic composition according to any of the preceding claims, wherein a dye and/or a chemical substance, in particular a pesticide, a drug or an insect repellent, is dissolved in the thixotropic composition and/or pigments, any particles having a diameter in the range of 1 to 100 µm, metal particles, nanocapsules and/or microcapsules are suspended in the thixotropic composition.

8. Thixotropic composition according to claim 7, wherein the metal particles are particles of a heavy metal, in particular copper, silver or zinc.

9. Thixotropic composition according to claim 8, wherein the metal particles are particles of silver having a diameter in the range of 1 to 100 µm and an arithmetic mean inner porosity of at least 65%, in particular between 65% and 90% or 95%.

10. Thixotropic composition according to claim 7, 8 or 9, wherein the metal particles are present in the thixotropic composition in a concentration range of 0.005% w/w to 0.5% w/w, in particular in a concentration range of 0.05% w/w to 0.2% w/w.

11. Thixotropic composition according to any of the preceding claims, wherein the metal particles are encapsulated metal particles or metal particles having an optical effect by having a ratio of surface area to volume of at least 5.

12. Thixotropic composition according to any of the preceding claims, wherein the solvent comprises or consists of water and a diol, in particular a butanediol or a propanediol, a triol, in particular glycerol, or a polyol.

13. Thixotropic composition according to claim 12, wherein the content of water in the composition is in a range of 80% w/w to 90% w/w or at least 85% w/w, in particular at least 95% w/w, in particular 95% w/w to 98% w/w.

14. Thixotropic composition according to any of the preceding claims, wherein the crystalline cellulose is partly depolymerized microcrystalline cellulose having CAS Registry Number 9004-34-6 and/or the starch glycolate is an alkaline starch glycolate, in particular sodium starch glycolate.

15. Thixotropic composition according to any of the preceding claims, wherein the alkali salt of the carboxymethyl cellulose is sodium carboxymethyl cellulose, in particular sodium carboxymethyl cellulose having CAS Registry Number 9004-32-4, and wherein the alkaline earth metal salt of the carboxymethyl cellulose is calcium carboxymethyl cellulose or magnesium carboxymethyl cellulose.

16. Thixotropic composition according to any of the preceding claims, wherein the thixotropic composition is formulated as a body care product, a sunscreen, a medicament, a spray for plants, a paint, a lacquer, an impregnant or another coating to be applied on a surface for coating that surface.

17. Spraying device containing the thixotropic composition according to any of the preceding claims, wherein content of water of the thixotropic composition in the spraying device is at least 85% w/w.

18. Use of the thixotropic composition according to any of claims 1 to 16 for the preparation of a medical cream, a wound treatment product, a body care product, a sunscreen or a cosmetic.

## Patentansprüche

1. Thixotrope Zusammensetzung, umfassend eine mikrokristalline Cellulose, ein Quell- oder Wasserbindemittel, ein Stärkeglykolat, ein Lösungsmittel und Carboxymethylcellulose oder ihr Alkalisalz oder ihr Erdalkalimetallsalz, wobei das Quell- oder Wasserbindemittel Glimmer ist, wobei das Lösungsmittel Wasser umfasst oder aus Wasser besteht, wobei der Wassergehalt der Zusammensetzung mindestens 70 Gew.-% beträgt, wobei das Gewichtsverhältnis von mikrokristalliner Cellulose zu Carboxymethylcellulose oder ihrem Alkalisalz oder ihrem Erdalkalimetallsalz im Bereich von 3 : 1 bis 11 : 1 liegt und/oder das Gewichtsverhältnis von mikrokristalliner Cellulose und Carboxymethylcellulose oder deren Alkalisalz oder deren Erdalkalimetallsalz zusammen zum Quell- oder Wasserbindemittel im Bereich von 3 : 1 bis 4 : 1 liegt.

2. Thixotrope Zusammensetzung nach Anspruch 1, wobei der Wassergehalt der Zusammensetzung mindestens 80 Gew.-% beträgt.

3. Thixotrope Zusammensetzung nach Anspruch 1 oder 2, wobei der Glimmer Glimmer mit der CAS-Registernummer 12001-26-2 ist.

4. Thixotrope Zusammensetzung nach Anspruch 1 oder 2, wobei der Glimmer ein Aluminium-Fluor-Magnesium-Natriumsilikat ist.

5. Thixotrope Zusammensetzung nach einem der vorhergehenden Ansprüche, wobei das Gewichtsverhältnis von mikrokristalliner Cellulose zu Carboxymethylcellulose oder deren Alkalisalz oder deren Erdalkalimetallsalz im Bereich von 4 : 1 bis 9 : 1 liegt und/oder das Gewichtsverhältnis von mikrokristalliner Cellulose und Carboxymethylcellulose oder deren Alkalisalz oder deren Erdalkalimetallsalz zusammen zum Quell- oder Wasserbindemittel im Bereich von 3,3 : 1 bis 3,5 : 1 liegt.

6. Thixotrope Zusammensetzung nach einem der vorhergehenden Ansprüche, wobei die thixotrope Zusammensetzung weiterhin eine Cellulose oder eine Alkylcellulose, insbesondere Methylcellulose, Ethylcellulose oder Ethylmethylcellulose, umfasst.

7. Thixotrope Zusammensetzung nach einem der vorhergehenden Ansprüche, wobei in der thixotropen Zusammensetzung ein Farbstoff und/oder eine chemische Substanz, insbesondere ein Pestizid, ein Arzneimittel oder ein Insektenschutzmittel, gelöst ist und/oder in der thixotropen Zusammensetzung Pigmente, beliebige Partikel mit einem Durchmesser im Bereich von 1 bis 100 µm, Metallpartikel, Nanokapseln und/oder Mikrokapseln suspendiert sind.

8. Thixotrope Zusammensetzung nach Anspruch 7, wobei die Metallpartikel Partikel eines Schwermetalls, insbesondere Kupfer, Silber oder Zink sind.

9. Thixotrope Zusammensetzung nach Anspruch 8 , wobei die Metallpartikel Silberpartikel mit einem Durchmesser im Bereich von 1 bis 100 µm und einer arithmetischen mittleren inneren Porosität von mindestens 65 %, insbesondere zwischen 65 % und 90 % oder 95 %, sind.

10. Thixotrope Zusammensetzung nach Anspruch 7 , 8 oder 9, wobei die Metallpartikel in der thixotropen Zusammensetzung in einem Konzentrationsbereich von 0,005 Gew.-% bis 0,5 Gew.-%, insbesondere in einem Konzentrationsbereich von 0,05 Gew.-% bis 0,2 Gew.-%, vorhanden sind.

11. Thixotrope Zusammensetzung nach einem der vorhergehenden Ansprüche, wobei die Metallpartikel eingekapselte Metallpartikel oder Metallpartikel sind, die einen optischen Effekt haben, indem sie ein Verhältnis von Oberfläche zu Volumen von mindestens 5 haben.

12. Thixotrope Zusammensetzung nach einem der vorhergehenden Ansprüche, **dadurch gekennzeichnet, dass** das Lösungsmittel Wasser und ein Diol, insbesondere ein Butandiol oder ein Propandiol, ein Triol, insbesondere Glycerin, oder ein Polyol umfasst oder daraus besteht.

13. Thixotrope Zusammensetzung nach Anspruch 12, wobei der Wassergehalt in der Zusammensetzung in einem Bereich von 80 Gew.-% bis 90 Gew.-% liegt oder mindestens 85 Gew.-%, insbesondere mindestens 95 Gew.-%, insbesondere 95 Gew.-% bis 98 Gew.-% beträgt.

14. Thixotrope Zusammensetzung nach einem der vorhergehenden Ansprüche, wobei die kristalline Cellulose teilweise depolymerisierte mikrokristalline Cellulose mit der CAS-Registernummer 9004-34-6 ist und/oder das Stärkeglycolat ein Alkalistärkeglycolat, insbesondere Natriumstärkeglycolat, ist.

15. Thixotrope Zusammensetzung nach einem der vorhergehenden Ansprüche, wobei das Alkalisalz der Carboxymethylcellulose Natriumcarboxymethylcellulose ist, insbesondere Natriumcarboxymethylcellulose mit der CAS-Registernummer 9004-32-4, und wobei das Erdalkalimetallsalz der Carboxymethylcellulose Calciumcarboxymethylcellulose oder Magnesiumcarboxymethylcellulose ist.

16. Thixotrope Zusammensetzung nach einem der vorhergehenden Ansprüche, wobei die thixotrope Zusammensetzung als ein Körperpflegemittel, ein Sonnenschutzmittel, ein Medikament, ein Spray für Pflanzen, eine Farbe, ein Lack, ein Imprägniermittel oder eine andere Beschichtung zum Auftragen auf eine Oberfläche zur Beschichtung dieser Oberfläche formuliert ist.

17. Sprühvorrichtung, die die thixotrope Zusammensetzung nach einem der vorhergehenden Ansprüche enthält, wobei der Wassergehalt der thixotropen Zusammensetzung in der Sprühvorrichtung mindestens 85 Gew.-% beträgt.

18. Verwendung der thixotropen Zusammensetzung nach einem der Ansprüche 1 bis 16 zur Herstellung einer medizinischen Creme, eines Wundbehandlungsmittels, eines Körperpflegemittels, eines Sonnenschutzmittels oder eines Kosmetikums.

## Revendications

1. Composition thixotrope comprenant une cellulose microcristalline, un agent gonflant ou de liaison à l'eau, un glycolate d'amidon, un solvant, et de la carboxyméthylcellulose ou son sel alcalin ou son sel métallique alcalino-terreux, dans laquelle l'agent gonflant ou de liaison à l'eau est le mica, dans laquelle le solvant comprend ou est constitué d'eau, dans laquelle une teneur en eau de la composition est d'au moins 70 % p/p, dans laquelle le rapport pondéral de cellulose microcristalline sur carboxyméthylcellulose ou son sel alcalin ou son sel métallique alcalino-terreux est dans la plage de 3/1 à 11/1 et/ou le rapport pondéral de cellulose microcristalline et de carboxyméthylcellulose ou son sel alcalin ou son sel métallique alcalino-terreux ensemble sur l'agent gonflant ou de liaison à l'eau est dans la plage de 3/1 à 4/1.

2. Composition thixotrope selon la revendication 1, dans laquelle la teneur en eau de la composition est d'au moins 80 % p/p.

3. Composition thixotrope selon la revendication 1 ou 2, dans laquelle le mica est du mica ayant le Numéro de registre CAS 12001-26-2.

4. Composition thixotrope selon la revendication 1 ou 2, dans laquelle le mica est un silicate d'aluminium fluoro-magnésium sodium.

5. Composition thixotrope selon l'une quelconque des revendications précédentes, dans laquelle le rapport pondéral de cellulose microcristalline sur carboxyméthylcellulose ou son sel alcalin ou son sel métallique alcalino-terreux est dans la plage de 4/1 à 9/1 et/ou le rapport pondéral de cellulose microcristalline et de carboxyméthylcellulose ou son sel alcalin ou son sel métallique alcalino-terreux ensemble sur l'agent gonflant ou de liaison à l'eau est dans la plage de 3,3/1 à 3,5/1.

6. Composition thixotrope selon l'une quelconque des revendications précédentes, dans laquelle la composition thixotrope comprend en outre une cellulose ou une alkylcellulose, notamment méthylcellulose, éthylcellulose ou éthylméthylcellulose.

7. Composition thixotrope selon l'une quelconque des revendications précédentes, dans laquelle un colorant et/ou une substance chimique, notamment un pesticide, un médicament ou un insectifuge, est dissous dans la composition thixotrope et/ou des pigments, toute particule ayant un diamètre dans la plage de 1 to 100 µm, des particules métalliques, nanocapsules et/ou microcapsules sont mis en suspension dans la composition thixotrope.

8. Composition thixotrope selon la revendication 7, dans laquelle les particules métalliques sont des particules d'un métal lourd, notamment de cuivre, d'argent ou de zinc.

9. Composition thixotrope selon la revendication 8, dans laquelle les particules métalliques sont des particules d'argent ayant un diamètre dans la plage de 1 à 100 µm et une porosité interne moyenne arithmétique d'au moins 65 %, notamment comprise entre 65 % et 90 % ou 95 %.

10. Composition thixotrope selon la revendication 7, 8 ou 9, dans laquelle les particules métalliques sont présentes dans la composition thixotrope dans une plage de concentration de 0,005 % p/p à 0,5 % p/p, notamment dans une plage de concentration de 0,05 % p/p à 0,2 % p/p.

11. Composition thixotrope selon l'une quelconque des revendications précédentes, dans laquelle les particules métalliques sont des particules métalliques encapsulées ou des particules métallique ayant un effet optique en ayant un rapport de surface sur volume d'au moins 5.

12. Composition thixotrope selon l'une quelconque des revendications précédentes, dans laquelle le solvant comprend ou est constitué d'eau et d'un diol, notamment d'un butanediol ou d'un propanediol, d'un triol, notamment de glycérol, ou d'un polyol.

13. Composition thixotrope selon la revendication 12, dans laquelle la teneur en eau dans la composition est dans une plage de 80 % p/p à 90 % p/p ou d'au moins 85 % p/p, notamment d'au moins 95 % p/p, notamment de 95 % p/p à 98 % p/p.

14. Composition thixotrope selon l'une quelconque des revendications précédentes, dans laquelle la cellulose cristalline est de la cellulose microcristalline partiellement dépolymérisée ayant le Numéro de registre CAS 9004-34-6 et/ou le glycolate d'amidon est un glycolate d'amidon alcalin, notamment le glycolate d'amidon sodique.

15. Composition thixotrope selon l'une quelconque des revendications précédentes, dans laquelle le sel alcalin de la carboxyméthylcellulose est la carboxyméthylcellulose de sodium, notamment la carboxyméthylcellulose de sodium ayant le Numéro de registre CAS 9004-32-4, et dans laquelle le sel métallique alcalino-terreux de la carboxyméthylcellulose est la carboxyméthylcellulose de calcium ou la carboxyméthylcellulose de magnésium.

16. Composition thixotrope selon l'une quelconque des revendications précédentes, dans laquelle la composition thixotrope est formulée en tant que produit de soin pour le corps, écran solaire, médicament, aérosol pour les plantes, peinture, laque, produit d'imprégnation ou autre enrobage devant être appliqué sur une surface pour l'enrobage de cette surface.

17. Dispositif de pulvérisation contenant la composition thixotrope selon l'une quelconque des revendications précédentes, dans lequel la teneur en eau de la composition thixotrope dans le dispositif de pulvérisation est d'au moins 85 % p/p.

18. Utilisation de la composition thixotrope selon l'une quelconque des revendications 1 à 16 pour la préparation d'une crème médicale, d'un produit de traitement des plaies, d'un produit de soin pour le corps, d'un écran solaire ou d'un produit cosmétique.
